# EUROPEAN PATENT APPLICATION

(11) **EP 3 741 837 A1**
(43) Date of publication of application: **25.11.2020**
(21) Application number: 20160598.7
(22) Date of filing: 03.03.2020
(51) Int. Cl.: C12G 1/02

(54) **FERMENTATION METHOD**

(30) Priority: 06.03.2019 IT 201900003235
(71) Applicant: Noform S.r.l., 30020 Meolo (VE) (IT)
(72) Inventor: CROSATO, REMO, 30020 MEOLO (IT)
(74) Representative: Citron, Massimiliano

(57) **Abstract**

A fermentation method is described for a vegetable liquid which, by fermenting inside a horizontal-axis (X) cylinder (10) of a rotary autoclave, produces a deposit of yeasts (90) on the bottom of the cylinder, comprising the steps of
(i) lifting some yeasts comprised in the deposit up to (i) causing them to move away and/or detach them from the inner surface of the cylinder so that they float in the liquid and then fall back to the bottom of the cylinder along a trajectory that joins two separate points of the inner surface of the cylinder.

## Description

The invention relates to a fermentation method for a vegetable liquid inside a rotary autoclave and a rotary autoclave to implement the method. The following description will refer by way of example to winemaking, a field in which the invention has proven to be particularly effective.

It is known that autoclaves are used for the fermentation of wine according to the Charmat method (or Martinotti method).

EP0246202A1 describes a horizontal-axis rotating drum equipped internally with a double cochlea to move the sediment of lees towards the frustoconical neck of the drum or towards its bottom. By performing a rotation of the autoclave, during the sparkling process, EP0246202A1 claims to simulate the manual movement of the bottles typical of the Champenois method.

It is not clear how the cochlea can actually achieve the desired effect, being it notoriously capable only of evacuating the lees from the tank. Unfortunately the sediments always remain between the coils of the cochlea and during the rotation of the drum they can move only along the cochlea accumulating in the conical part, from where they will then be evacuated through the emptying hatch located at the end of the cone. In other words, the sediments can only slide on the wall inside the drum, while for optimal fermentation it is needed to distribute the yeasts as uniformly as possible in the inner volume of the drum, as if they fermented in a bottle turned by hand.

EP0246202A1 does not mention the problem of mixing yeasts.

Furthermore, the frustoconical drum of EP0246202A1 is complex to build, expensive and requires high thicknesses of material in the pointed part.

Another disadvantage of EP0246202A1 is that the drum is operated with means which directly engage the external surface of the drum, subtracting space from the thermoregulation pockets and thus preventing an effective thermoregulation, indispensable quality in an autoclave because the fermentation is very sensitive to the correct reduction of temperature.

The present invention has as main object to improve this state of the art.

Another object is to propose an autoclave that improves the effects obtainable from that in EP0246202A1, in particular an autoclave that mixes the yeasts better.

In general, relative terms such as *vertical, up, top* and *bottom* are meant as referred to the autoclave as in use.

A first aspect of the invention relates to a fermentation method for a vegetable liquid which, by fermenting inside a horizontal-axis cylinder of a rotary autoclave, produces a deposit of yeast on the bottom of the cylinder,
comprising the steps of
(i) lifting the yeasts comprised in the deposit up to
(ii) making them move away (and/or detach them) from the internal surface of the cylinder so that the yeasts float in the liquid and then fall back to the bottom of the cylinder along a trajectory that joins two separate points of the inner surface of the cylinder.

In this way during the precipitation the yeasts pass through a large part of the fermenting liquid and distribute in it with better uniformity.

The said trajectory joins two separate points of the inner surface of the cylinder and, preferably remaining at a certain distance from the inner surface of the cylinder, passes through a part of the inner volume of the cylinder. E.g. the trajectory passes through or near the axis of the cylinder, or, viewed along the axis of the cylinder, intersects a radius of a cross-section of the cylinder.

The step (i) and (ii) may be carried out e.g.
by sucking the sediments from the bottom and injecting them on top of the cylinder, or
by mechanically lifting the sediments from the bottom of the cylinder to spread them inside the cylinder.

According to a preferred, simple and effective step of the method, the steps (i) and (ii) are obtained by rotating the cylinder about the axis.

According to a preferred step of the method, which improves the distribution of yeasts, yeasts comprised in the deposit are forced to slide over elements or walls that extend cantilevered from the inner surface of the cylinder, so as to move them towards the axis or center of the cylinder. The step of forcing the yeasts to slide over said elements or walls preferably and advantageously occurs by rotating the cylinder about the axis.

A second aspect of the invention relates to a rotary autoclave for the fermentation of a vegetable liquid, comprising
a frame,
a cylinder with horizontal axis for containing the vegetable liquid to be fermented until it produces a deposit of yeasts on the bottom of the cylinder,
the cylinder being mounted on the frame so that it can rotate about the axis,
means for lifting the yeasts comprised in the deposit up to make them move away (and/or detach them) from the inner surface of the cylinder in order to make them float in the liquid and then fall to the bottom of the cylinder along a trajectory that joins two separated points of the inner surface of the cylinder.

Said means may comprise e.g.
an aspirator to suck the sediments from the bottom and inject them on top of the cylinder, or
a lifting device for mechanically lifting the sediments from the bottom of the cylinder and spread them inside the cylinder, or
one or more injectors of inert or oxygenating gas adapted for, in addition to let the fermenting mass interact with said injected gas, moving the mass itself and the yeasts, increasing their multiplication capacity or facilitating the elimination of odors deriving from bad fermentations. In fact, sometimes together with desired yeasts during alcoholic fermentations or re-fermentations, yeasts are formed that produce unwanted odors or flavors. The variant of the invention which comprises gas injection greatly improves the final product.

According to a preferred embodiment of the autoclave, said means comprise rigid or flexible elements or walls which extend cantilevered from the inner surface of the cylinder so that, during a rotation of the cylinder, yeasts belonging to the deposits are lifted and forced to slide over said elements or walls to move towards the axis of the cylinder.

Said elements or walls may be e.g. of septa, wedges, blades or grids.

The autoclave then allows bringing the yeasts previously lying on the bottom of the cylinder at a greater height, in particular at a greater height than the axis of the cylinder. Thus, when the yeasts fall by gravity they pass through all the liquid present between the point of detachment from said elements or walls and the bottom, and the diffusion of yeasts in the fermenting liquid improves.

Preferably said elements or walls extend radially with respect to the inner surface of the cylinder, e.g. along a chord that joins two points of the imaginary circumference defining the cross-section of the cylinder. In particular said elements or walls extend on a plane passing through the cylinder axis, or on a diametrical plane of the cylinder.

Preferably said elements or walls protrude from the inner surface of the cylinder extending parallel to the axis of the cylinder. The length of said elements or walls may or may not coincide with the horizontal length of the cylinder.

Preferably said elements or walls are uniformly and/or symmetrically distributed on the inner surface of the cylinder, e.g. with polar symmetry with respect to the axis of the cylinder.

Preferably one or each of said elements or walls protrudes from the inner surface of the cylinder for a length between 5 and 40 cm, in particular between 5 and 20 cm.

Preferably the cylinder at its ends has a bulging convexity, to avoid the problems, the cost and the weight of a frustoconical shape.

Preferably, the autoclave comprises an electric motor for setting the cylinder into rotation, the motor being coupled to the cylinder only at one end of the cylinder. In this way the side surface of the cylinder remains freer for the application of thermoregulation pockets.

A third aspect of the invention relates to the use of an aforementioned rotary autoclave for
lifting yeasts deposited on the bottom of the cylinder up to a level equal to or greater than that of the cylinder's rotation axis, and then
letting the raised yeasts precipitate by gravity into the internal volume of the cylinder.

A fourth aspect of the invention relates to a fermentation method with the steps of
lifting yeasts, deposited on the bottom of the cylinder of a rotary autoclave, up to a level equal to or greater than that of the cylinder's rotation axis, and then
letting the raised yeasts precipitate by gravity into the internal volume of the cylinder.

The above step of lifting yeasts preferably takes place by rotating the cylinder about the axis.

A preferred embodiment of the invention will now be described with reference to the attached drawing, wherein
fig. 1 shows a side view of an autoclave according to the invention;
fig. 2 and 3 show a cross-sectional view of the autoclave of fig. 1 in two operational configurations.

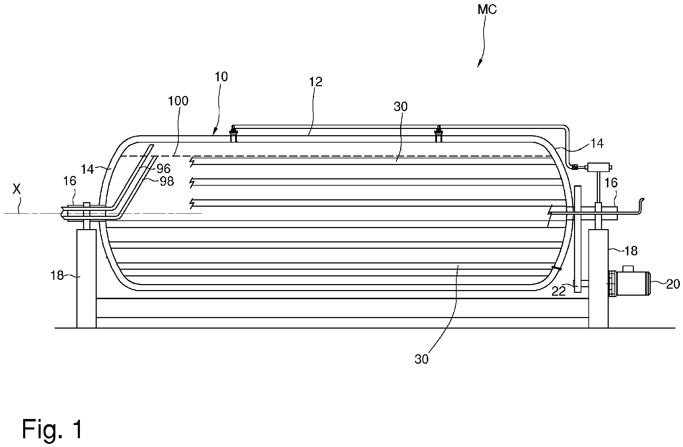

An autoclave MC comprises (fig. 1) a containing cylinder which is rotatably supported by a frame 18 about a horizontal axis X.

The containing cylinder 10 is formed by a hollow cylindrical shell 12, which constitutes the side wall thereof and which is closed at the ends by two convex walls 14. In the containing cylinder 10 e.g. some crushed material or must is loaded, up to a level 100, to ferment. Suitably a second outer shell allows containing the (optional) insulation which best guarantees the maintenance of the mass temperature, cooled or heated according to the related needs of alcoholic fermentation.

The center of the convex walls 14 is integral with two axial shafts 16 which rotatably support the containing cylinder 10 on the frame 18. A motor + gearmotor 20 is connected, via a transmission 22, to a shaft 16 for rotating the cylinder 10. The motor/gearmotor may also be connected directly to the shaft, without transmission.

On the inner surface of the shell 12 there are fins 30 (only some numbered) mounted cantilevered, which protrude from such surface and face the X axis. The fins 30 preferably run the entire inner surface of the shell 12 parallel to the X axis and/or are evenly distributed with polar symmetry around the X axis.

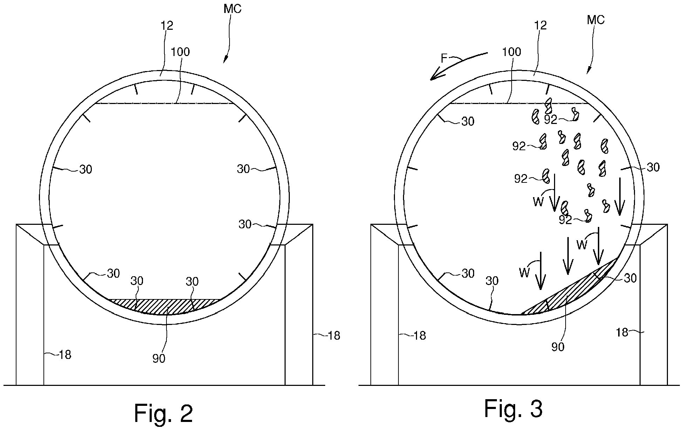

With reference to figures 2 and 3 the operation of the autoclave MC is illustrated.

During fermentation, on the bottom of the shell 12 yeasts 90 naturally accumulate and distribute between some fins 30.

The motor 20 is periodically activated to rotate the shell 12 (fig. 3, arrow F). Thus, the fins 30 drag the yeasts 90 and lift them about the X axis up to a height at which the raised yeasts slide by gravity over the fins 90 towards the center of the shell 12. The slipped yeasts (indicated with 92) then detach from the fins 90 and fall by gravity (see arrows W) towards the bottom of the shell 12. During the fall the yeasts 92 pass through and mix in the liquid mass contained in the cylinder 10.

Preferably the fins 30 raise the yeasts 90 to a level higher than that of the X axis.

As a variant, the cylinder 10 may also have a different shape from the illustrated one.

Means equivalent to the fins 30 may be e.g. wedges with vertex facing the inside of the cylinder 10. The fins 30 are e.g. flat or planar, or even concave. The activation of the motor 20 may follow various timings, e.g. slowly with continuity or with stop periods.

Fig. 1 also shows an optional but advantageous solution for the autoclave MC.

A shaft 16 is internally crossed by two pipes 96, 98 which open inside the cylinder 10. The tubes 96, 98 run vertically towards the top of the cylinder 10 and end at different heights. The pipe 98 is shorter than the pipe 96. The pipe 98 serves as "overflow" control, i.e. to establish the maximum level 100 of liquid inside the cylinder 10. The pipe 96, which by construction opens above the level 100, serves to evacuate fermentation gas from the cylinder 10.

The advantages of this configuration are e.g. that total filling of the tank is prevented to provide a "cushion" of fermenting gas under pressure. This cushion allows a controlled formation of fermentative CO₂ in the steps of depressurization or pressure jumps.

## Claims

1. Fermentation method for a vegetable liquid which, by fermenting inside a horizontal-axis (X) cylinder (10) of a rotary autoclave, produces a deposit of yeasts (90) on the bottom of the cylinder, comprising the steps of
(i) lifting some yeasts comprised in the deposit up to (i) causing them to move away and/or detach them from the inner surface of the cylinder so that they float in the liquid and then fall back to the bottom of the cylinder along a trajectory that joins two separate points of the inner surface of the cylinder.

2. Method according to claim 1, wherein step (i) and (ii) is carried out by sucking the deposited yeasts from said bottom and injecting them at the top of the cylinder, or by mechanically lifting the deposited yeasts from the bottom of the cylinder to spread them inside the cylinder.

3. Method according to claim 1 or 2, in which step (i) and (ii) is performed by rotating the cylinder about the axis (X).

4. Method according to claim 1 or 2 or 3, wherein the yeasts comprised in the deposit are forced to slide over elements or walls (30) that extend cantilevered from the inner surface of the cylinder, so as to move them towards the axis or the center of the cylinder.

5. Rotary autoclave (10) for the fermentation of a vegetable liquid, comprising:
a frame (18) and a cylinder (12) with horizontal axis (X) for containing the vegetable liquid to be fermented until it produces a deposit of yeasts on the bottom of the cylinder,
the cylinder being mounted on the frame so as to be able to rotate about the axis (X),
means (30) for lifting the yeasts comprised in the deposit until they are moved away and/or detached from the inner surface of the cylinder in order to make them float in the liquid and then precipitate on the bottom of the cylinder along a trajectory which joins two separate points of the inner surface of the cylinder.

6. Rotary autoclave according to claim 5, wherein said means comprise an aspirator for sucking the deposited yeasts from the bottom and injecting them at top of the cylinder.

7. Rotary autoclave according to claim 5 or 6, wherein said means comprise a lifting device (30) for mechanically lifting the deposited yeasts from the bottom of the cylinder and spreading them inside the cylinder.

8. Rotary autoclave according to claim 5 or 6 or 7, wherein said means comprise one or more inert or oxygenating gas injectors adapted to move the liquid and the yeasts.

9. Rotary autoclave according to claim 5 or 6 or 7 or 8, wherein said means comprise rigid or flexible elements or walls (30) which extend cantilevered from the inner surface of the cylinder so that, during a rotation of the cylinder, yeasts belonging to the deposit are lifted and forced to slide over said elements or walls to move towards the cylinder's axis.

10. Use of a aforesaid rotary autoclave for lifting yeasts deposited on the bottom of the cylinder up to height equal to or greater than that of the rotation axis of the cylinder, and then letting the raised yeasts precipitate by gravity within the inner volume of the cylinder.
